# EUROPÄISCHE PATENTANMELDUNG

(11) **EP 0 670 298 A1**
(43) Veröffentlichungstag der Anmeldung: **06.09.1995**
(21) Anmeldenummer: 95102152.6
(22) Anmeldetag: 16.02.1995
(51) Int. Cl.: C07C 43/215, C08G 77/38, C09K 19/40

(54) **Materialien auf Stilbenbasis, ihre Herstellung und Verwendung**

(30) Priorität: 18.02.1994 DE 4405220; 17.03.1994 DE 4409207
(71) Anmelder: Consortium für elektrochemische Industrie GmbH, D-81379 München (DE)
(72) Erfinder: Weitzel, Hans-Peter, Dr., D-84571 Reischach (DE); Leigeber, Horst, D-82041 Oberhaching (DE); Boldt, Peter, Prof. Dr., D-38106 Braunschweig (DE); Leupold, Jobst, D-38118 Braunschweig (DE)

(57) **Zusammenfassung**

Die Erfindung betrifft Materialien der Formel I: 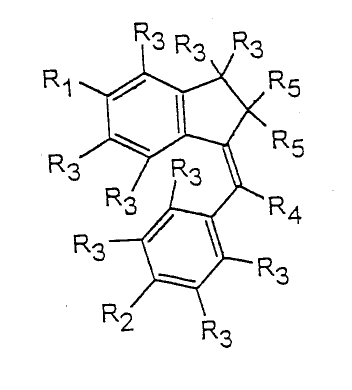
wobei
R₁, R₂, und R₃ jeweils unabhängig voneinander gleich oder verschieden sein können und Wasserstoffatom, Halogenatom, Hydroxygruppe, Nitrogruppe, Cyanogruppe oder einwertiger, gegebenenfalls substituierter organischer Rest bedeuten und
R₄ Wasserstoffatom, Halogenatom, Nitrogruppe, Cyanogruppe oder einwertiger, gegebenenfalls substituierter organischer Rest bedeutet und
R₅ gleich oder verschieden sein kann und Halogenatom, Hydroxygruppe, Nitrogruppe, Cyanogruppe oder einwertiger, gegebenenfalls substituierter organischer Rest bedeutet.

## Beschreibung

Die Erfindung betrifft Materialien auf Stilbenbasis, ihre Herstellung und Verwendung zur Herstellung von mittels Licht strukturierbaren Materialien.

Gängige Materialien, die sich mit Licht im sichtbaren Bereich strukturieren lassen sind beispielsweise Azofarbstoffe. Anwendungsgebiete für solche Materialien sind u.a. Informationsspeicherung und mittels Licht strukturierbare optische Elemente wie beispielsweise holographische, optische Elemente.

Für verschiedene Anwendungen sind farbige Materialien ungeeignet, da die Eigenfarbe eine Einschränkung des nutzbaren Spektralbereiches darstellt. Für farblose optische Elemente werden vorzugsweise Materialien ohne Absorptionsbanden im sichtbaren Spektralbereich aber mit Absorptionsbanden im UV oder IR Bereich verwendet.

Im nahen Infrarotbereich werden z.B. Merocyanine, Fulgide und Spiropyrane verwendet. Bei den bekannten Materialien für optische Elemente mit Absorptionsbanden im infraroten Bereich ist eine hohe Zahl an Schreib-Lösch-Cyclen nicht gewährleistet, da die Farbstoffe nicht langzeitstabil sind (Fabian, Chem. Rev. 1992, 1197). Außerdem besitzen viele dieser sogenannten Infrarotfarbstoffe zusätzlich kürzerwellige Absorptionsbanden und erscheinen deshalb nicht völlig farblos.

Farblose Devices können im Prinzip auch durch Materialien mit Absorptionsbanden ausschließlich im nahen UV Bereich von 250 bis 380 nm realisiert werden. Solche Devices besitzen den Vorteil, daß gespeicherte Information nicht durch sichtbares Licht wieder gelöscht wird. Außerdem ist die erreichbare Speicherdichte bei Verwendung kürzerwelligen Lichtes zum Einschreiben der Information deutlich höher. Die Auswahl der Farbstoffe ist hier jedoch sehr beschränkt. Als photochrome Verbindungen kommen hier in erster Linie Stilbene in Frage. Stilbenoide Verbindungen besitzen aber einige schon seit langem bekannte Nebenreaktionen, wie z.B. Cyclisierung zu Dihydrophenanthrenderivaten (mit anschließender Oxidation zu Phenanthren) oder Dimerisierung zu Cyclobutanen. Sie können daher bislang nicht für optische Informationsspeicherung benutzt werden (L. Feringa, Tetrahedron, 1993, 8267-8310, H. Meier, Angewandte Chemie, Vol. 31, Nr. 11, November 1992, S. 1399-1540).

Aufgabe der Erfindung war es, Materialien auf Stilbenbasis, die keine irreversiblen photochemischen Reaktionen zeigen, zur Verfügung zu stellen.

Eine weitere Aufgabe der Erfindung war es, mittels elektromagnetischer Wellen strukturierbare Polymere enthaltend Materialien auf Stilbenbasis zur Verfügung zu stellen.

Die Aufgabe wird gelöst durch Materialien der Formel I: 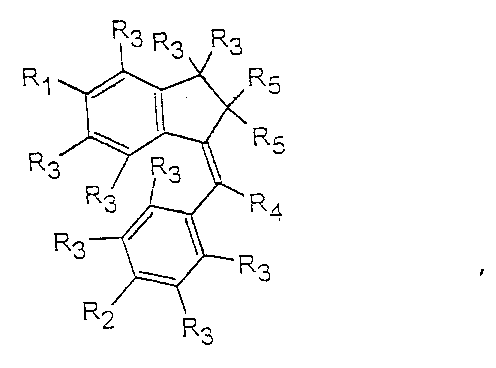
wobei
R₁, R₂, und R₃ jeweils unabhängig voneinander gleich oder verschieden sein können und Wasserstoffatom, Halogenatom, Hydroxygruppe, Nitrogruppe, Cyanogruppe oder einwertiger, gegebenenfalls substituierter organischer Rest bedeuten und
R₄ Wasserstoffatom, Halogenatom, Nitrogruppe, Cyanogruppe oder einwertiger, gegebenenfalls substituierter organischer Rest bedeutet und
R₅ gleich oder verschieden sein kann und Halogenatom, Hydroxygruppe, Nitrogruppe, Cyanogruppe oder einwertiger, gegebenenfalls substituierter organischer Rest bedeutet.

Vorzugsweise handelt es sich bei den oben genannten gegebenenfalls substituierten organischen Resten um Kohlenwasserstoffreste mit 1 bis 18 Kohlenstoffatomen, die gegebenenfalls durch Halogenatome, Ethergruppen, Estergruppen, Ketogruppen, Epoxygruppen, oder Cyanogruppen substituiert sind.

Beispiel für solche Kohlenwasserstoffreste sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest, Dodecylreste, wie der n-Dodecylrest; Octadecylreste, wie der n-Octadecylrest; Alkenylreste, wie Vinyl-, Butenyl-, Pentenyl-, Hexenyl-, Heptenyl-, Oktenyl-, Oktadienyl-, Decenyl-, Dodecenyl-, Hexadecenyl-, und der Allylrest; Alkoxyreste, wie der Methoxy-, Ethoxy-, n-Propoxy-, Isopropoxy-, n-, sec.- und tert.-Butoxyrest, Pentoxy-, Hexoxy-, Oktoxy-, Decoxy-, Hexadecoxyrest; Alkenoxyreste, wie der Allyloxyrest, Butenyloxy-, Pentenyloxy-, Hexenyloxy-, Oktenyloxy-, Decenyloxy- und Hexadecenyloxyrest; Cycloalkylreste, wie Cyclopentyl-, Cyclohexyl-, Cycloheptylreste und Methylcyclohexylreste; Cycloalkenylreste wie Cyclopentenyl-, Cyclohexenyl- und Cycloheptenylreste; Cholestanreste; der Cholesterylrest; Fluor-, Chlor- oder Bromatome; Wasserstoffatome; Hydroxyl-, Nitril-, (Meth)acryloxy-, (Meth)acryloxyethylenoxy-, (Meth)acryloxydi(ethylenoxy)-, (Meth)acryloxytri(ethylenoxy)- und Trimethylsilyl-, Triethylsilylgruppen; Arylreste, wie der Phenyl-, Naphthyl-, Anthryl- und Phenanthrylrest; Alkarylreste, wie o-, m-, p-Tolylreste, Xylylreste und Ethylphenylreste, Aralkylreste, wie der Benzylrest, der α- und der β-Phenylethylrest.

Beispiel für substituierte Kohlenwasserstoffreste sind Cyanalkylreste, wie der β-Cyanethylrest, und halogenierte Kohlenwasserstoffreste, beispielsweise Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m-, und p-Chlorphenylrest.

Bevorzugt bedeuten in Formel I:
R₁, R₂, R₃ jeweils unabhängig voneinander Wasserstoffatom, Alkyl-, Alkenyl-, Alkoxy- oder Alkenoxyrest,
R₄ Wasserstoffatom oder Alkylrest,
R₅ Alkylrest.

Besonders bevorzugt bedeuten in Formel I:
R₁ Alkenoxyrest
R₂ Wasserstoffatom, Alkyl- oder Alkoxyrest
R₃ Wasserstoffatom
R₄ Wasserstoffatom
R₅ Methylrest.

Die erfindungsgemäßen Materialien der Formel I zeigen weder Dimerisierung noch Cyclisierung, wie sie bei bekannten Materialien auf Stilbenbasis auftreten.

Die einzige zu beobachtende photochemische Umwandlung ist die gewünschte cis/trans Isomerisierung.

Die erfindungsgemäßen Materialien lassen sich nach an sich bekannten Verfahren herstellen, wie sie beispielsweise in J. March, Advanced Org. Chemistry, John Wiley & Son, 3. Ausgabe, 1985, S. 816, S. 416 beschrieben sind.

Vorzugsweise werden die erfindungsgemäßen Materialien durch Alkylierung von ggf. substituierten 1-Indanonen mit Alkylhalogeniden, anschließender Grignardreaktion mit ggf. substituierten Benzylmagnesiumhalogeniden und folgender saurer Aufarbeitung hergestellt.

Bei den im erfindungsgemäßen Verfahren eingesetzten 1-Indanonen handelt es sich vorzugsweise um solche der Formel II 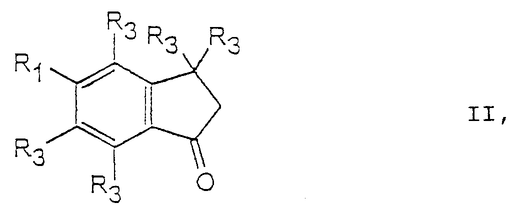
wobei R₁ und R₃ die für Formel I genannten Bedeutungen haben.

Vorzugsweise werden im erfindungsgemäßen Verfahren methylsubstituierte 1-Indanone oder 5-Alkoxy substituierte 1-Indanone eingesetzt.

Besonders bevorzugt wird 5-Methoxy-1-indanon eingesetzt.

Die im erfindungsgemäßen Verfahren einsetzbaren 1-Indanone sind käuflich erhältlich oder können nach bekannten Verfahren, wie beispielsweise in J. March, Advanced Org. Chemistry, John Wiley & Son, 3. Ausgabe, 1985, S. 486, beschrieben, hergestellt werden.

Bei den im erfindungsgemäßen Verfahren eingesetzten ggf. substituierten Benzylmagnesiumhalogeniden handelt es sich vorzugsweise um solche der Formel III 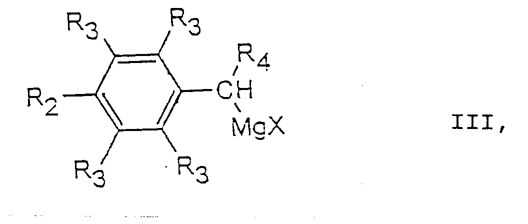
wobei R₂, R₃ und R₄ die für die Formel I genannten Bedeutungen haben.

Vorzugsweise werden im erfindungsgemäßen Verfahren Benzylmagnesiumhalogenide oder 4-substituierte Benzylmagnesiumhalogenide eingesetzt.

Besonders bevorzugt wird Benzylmagnesiumchlorid oder 4-Alkoxy-Benzylmagnesiumchlorid eingesetzt.

Die im erfindungsgemäßen Verfahren eingesetzten ggf. substituierten Benzylmagnesiumhalogeniden sind käuflich erhältlich oder können nach bekannten Verfahren, wie beispielsweise in J. March, Advanced Org. Chemistry, John Wiley & Son, 3. Ausgabe, 1985, S. 816 beschrieben, hergestellt werden.

Vorzugsweise erfolgt die Alkylierung des ggf. substituierten 1-Indanons mit Alkylhalogeniden in einem geeigneten Lösungsmittel wie beispielsweise tert.-Butanol oder Tetrahydrofuran, wobei vorzugsweise pro Mol 1-Indanon 3 bis 4 Mol Alkylhalogenid sowie 1,0 - 1,1 Mol tert.-Butanol eingesetzt werden.

Vorzugsweise wird die Umsetzung bei einer Temperatur zwischen 20 und 80°C unter Atmosphärendruck durchgeführt. Ggf. können die Substituenten noch in an sich bekannter Weise in jeweils gewünschtem Maß modifiziert werden. So ist es beispielsweise möglich, Ethersubstituenten durch Behandlung mit BBr₃ (2 Mol/ Mol Ether) in Dichlormethan zu spalten und den freiwerdenden Alkohol weiter zu derivatisieren.

Aus dem so erhaltenen modifizierten 1-Indanon werden anschließend durch Grignard Reaktion mit ggf. modifiziertem Benzylmagnesiumhalogenid die erfindungsgemäßen Substanzen auf Stilbenbasis hergestellt. Dazu werden pro Mol modifiziertes 1-Indanon vorzugsweise 2 bis 3 Mol ggf. substituiertes Benzylmagnesiumhalogenid eingesetzt. Die Reaktion erfolgt vorzugsweise bei einer Temperatur zwischen 20 und 70°C bei Normaldruck. Durch anschließende Säureaufarbeitung zur Eliminierung des primär entstehenden Alkohols und chromatographische Reinigung erhält man die erfindungsgemäßen Produkte.

Die Auftrennung der erfindungsgemäßen Produkte in die Isomeren kann, falls erwünscht, nach an sich bekannten Methoden wie beispielsweise chromatographischen Verfahren erfolgen.

Die erfindungsgemäßen Materialien auf Stilbenbasis gemäß Formel I lassen sich durch Austausch eines der einwertigen Reste R₁ bis R₅ durch einen zweiwertigen organischen Rest an beliebige Polymerrückgrate anknüpfen.

Vorzugsweise erfolgt die Anknüpfung über Austausch eines der einwertigen Reste R₁ bis R₃ besonders bevorzugt des einwertigen Restes R₁ gegen einen zweiwertigen organischen Rest.

Es zeigte sich überraschenderweise, das sich solche Polymere besonders gut durch elektromagnetische Wellen strukturieren lassen.

Daher wird die weitere Aufgabe der Erfindung gelöst durch Polymere, die mindestens einen Stilbenrest der Formel I aufweisen, bei dem einer der einwertigen Reste R₁ bis R₅, bevorzugt einer der Reste R₁ bis R₃ besonders bevorzugt der Rest R₁ gegen einen zweiwertigen organischen Rest ausgetauscht ist.

Die erfindungsgemäßen Polymere besitzen vorzugsweise ein Polymerrückgrat, welches aus einem oder mehreren der Monomere ausgewählt aus der Gruppe Acrylate, Methacrylate, Vinylether, Vinylester, Chloracrylate, Cyanacrylate, Styrole, α-Methylstyrol und/oder aus cyclischen oder linearen Polysiloxanen aufgebaut ist, an welches als Seitenkette das Material auf Stilbenbasis gemäß Formel I durch Austausch eines der einwertigen Reste R₁ bis R₅ durch einen zweiwertigen organischen Rest R₆ angeknüpft ist.

Als zweiwertige organische Reste R₆ sind beispielsweise C₁ bis C₁₈ Alkylen- oder C₁ bis C₁₈ Alkoxylengruppen geeignet.

Insbesondere sind C₂ bis C₅ oder C₁₁ Alkylen- oder C₂ bis C₅ oder C₁₁ Alkoxylengruppen als zweiwertige organische Reste geeignet.

Bevorzugt handelt es sich bei dem Polymerrückgrat um ein Seitenkettenpolymer, mit flüssigkristallinen Eigenschaften.

Geeignete Seitenkettenpolymere mit flüssigkristallinen Eigenschaften sind beispielsweise in (Chem. Phys. Macromol; 1991, 211-271) beschrieben.

Besonders bevorzugt handelt es sich bei dem Polymerrückgrat um cyclische Polysiloxane, mit flüssigkristallinen Eigenschaften.

Geeignete cyclische Polysiloxane mit flüssigkristallinen Eigenschaften sind beispielsweise in US-5,211,877 und US-4,410,570 beschrieben.

Ggf. kann das erfindungsgemäße Polymer weitere mesogene Gruppen wie beispielsweise Derivate des Cyclohexans, wie Cyclohexylcarbonsäurecyclohexylester, Cyclohexylcarbonsäurephenylester, Cyclohexylphenylether, Cyclohexylbenzole, Dicyclohexylderivate, Derivate des Stilbens, Benzoesäurephenylester und seine Derivate, Steroide, wie Cholesterin, dessen Derivate, wie Cholesterinester, Cholestan und dessen Derivate, Benzylidenaniline, Azobenzol und seine Derivate, Azoxybenzol und dessen Derivate, Alkyl- und Alkoxyderivate von Biphenyl, Schiff'sche Basen oder nichtmesogene Gruppen oder photoreaktive Gruppen wie beispielsweise Azogruppen enthalten.

Das Polymerrückgrat kann nach bekannten Verfahren, wie sie beispielsweise in Makromol. Chem., Rapid Commun. 4, (1983), 795-799 beschrieben, sind hergestellt werden. Anschließend erfolgt die Anknüpfung des erfindungsgemäßen Stilbenderivates mittels an sich bekannter Verfahren, wie sie beispielsweise in Polym Bull. 1991, 27 (1), 37-40 beschrieben sind.

Die Herstellung des erfindungsgemäßen Polymers kann jedoch auch durch gleichzeitige Polymerisation des Polymerrückgrats und Anknüpfung des erfindungsgemäßen Stilbenderivates erfolgen. Dies ist beispielsweise wie in H. Finkelmann, Thermotropic Liquid Crystals, ed. G.W. Gray, Wiley Chichester CRAC Series Vol. 22, Chapter 6, 1987, S. 145-170 beschrieben, durchführbar.

Die Wahl geeigneter Polymerrückgrate und Copolymerzusammensetzungen ermöglicht die Herstellung von Materialien, die durch ultraviolettes Licht strukturiert werden können.

Auf Grund ihrer guten Strukturierbarkeit mittels elektromagnetischer Wellen eignen sich die erfindungsgemäßen Polymere insbesondere zur Herstellung von strukturierbaren optischen Elementen. Die Herstellung solcher Elemente kann in an sich bekannter Weise, wie beispielsweise bei DE 42 06 089 oder DE 41 37 943 beschrieben, erfolgen.

Bei den erfindungsgemäßen optischen Elemente handelt es sich beispielsweise um holographische optische Elemente wie holographische Gitter, holographische Linsen oder holographische Prismen. Solche Anwendungen sind beispielsweise in Holographic Recording Materials, Ed. H.M. Smith, Springer Verlag, 1977, Chapter 3.5, S. 97-99, beschrieben.

Zur weiteren Erläuterung der Erfindung dienen die folgenden Beispiele:

### Beispiel 1

### Synthese von 5-Allyloxy-2,2-dimethyl-1-(phenyl)methylenindan

a) 2,2-Dimethyl-5-methoxyindan-1-on
   5g (31 mmol) 5-Methoxyindan-1-on (Aldrich GmbH, Steinheim) und 17,5 g (123 mmol) Methyljodid wurden unter Stickstoffatmosphäre in 100 ml t-Butanol gelöst. Innerhalb von 45 Minuten wurden 13,8 g (123 mmol) Kalium-t-butanolat zugegeben. Das Reaktionsgemisch färbte sich dabei braun. Ein weißer Niederschlag wurde gebildet. Nach Beendigung der Zugabe wurde 1 h unter Rückfluß zum Sieden erhitzt. Das erkaltete Gemisch wurde auf 300 ml Wasser gegossen und 2 mal mit je 50 ml Dichlormethan extrahiert. Nach dem Trocknen über Magnesiumsulfat wurde das Lösungsmittel entfernt. Man erhielt das hellbraune, ölige Reaktionsprodukt in quantitativer Ausbeute.
b) 2,2-Dimethyl-5-hydroxyindan-1-on
   Zu einer Lösung von 6,1 g (32 mmol) der Verbindung aus a) in 100 ml Dichlormethan wurden unter Eiskühlung 64 ml einer 1M BBr₃-Lösung in Dichlormethan getropft und 96 Stunden bei Raumtemperatur gerührt. Danach wurde die dunkelbraune Reaktionslösung auf 200 ml Wasser gegossen und zwei mal mit je 50 ml Ether extrahiert. Die Etherphase wurde zwei mal mit 1 N Natronlauge ausgeschüttelt. Die alkalische wässrige Phase wurde dann mit verd. HCl angesäuert (pH 1) und wiederum zwei mal mit je 50 ml Ether extrahiert. Nach Filtration durch Kieselgel wurde der Ether abrotiert und man erhielt 4,6 g (26 mmol) eines hellbeigen Feststoffs.
c) 5-Allyloxy-2,2-dimethylindan-1-on
   4,6g (26 mmol) der Verbindung b), 6,3g (52mmol) Allylbromid und 14,4 g (103 mmol) Kaliumcarbonat wurden in 200 ml Aceton 2-3 h unter Rückfluß gekocht. Die Umsatzkontrolle erfolgte mit Dünnschichtchromatographie. Nach Abschluß der Umsetzung ließ man den Ansatz abkühlen. Anschließend wurde der Feststoff abfiltriert und mit 50 ml Aceton gewaschen. Beide Acetonfiltrate wurden vereinigt und das Aceton mittels eines Rotationsverdampfers entfernt. Man erhielt 5,5 g (25 mmol) eines hellbraunen Öls.
d) 5-Allyloxy-2,2-dimethyl-1-(phenyl)methylenindan
   Durch Zutropfen von 1 ml Brom und anschließend von 3,42g (27 mmol) Benzylchlorid zu 0,66g (27 mmol) Magnesiumspänen in 50 ml trockenem Ether und einstündigem Sieden unter Rückfluß wurde eine Lösung von Benzylmagnesiumbromid hergestellt. Nach dem Abkühlen wurde eine Lösung von 2 g (9,3 mmol) der Verbindung c) in 50 ml trockenem Ether zugetropft und 2 h unter Rückfluß zum Sieden gebracht. Danach wurde Eis zugegeben und 30 Minuten gerührt. Anschließend wurde unter Rühren verdünnte Salzsäure zugefügt bis sich der vorhandene Feststoff völlig gelöst hatte. Die Phasen wurden getrennt und die wässrige Phase zweimal mit je 50 ml Ether extrahiert. Die vereinigten Etherextrakte wurden mit Natriumhydrogencarbonatlösung entsäuert (pH 7), mit Wasser gewaschen und über Natriumsulfat getrocknet. Anschließend wurde der Ether abdestilliert.
   Der hellgelbe, ölige Rückstand wurde in 200 ml Benzol gelöst und nach Zugabe von 10 mg p-Toluolsulfonsäure für 4 Stunden auf 90°C erhitzt. Die abgekühlte Lösung wurde über Kieselgel filtriert und das Benzol unter vermindertem Druck (15 mbar) abdestilliert. Das hellgelbe Rohprodukt wurde im Hochvakuum getrocknet. Die weitere Reinigung erfolgt durch Plattenchromatographie (SiO₂/Petrolether). Die Produktfraktion (R_{f} = 0,27) wurde mit Methylenchlorid eluiert. Nach Entfernung des Methylenchlorids durch Destillation wurde das Produkt wieder in 50 ml Petrolether gelöst und durch neutrales Aluminium-oxid (Aktivitätsstufe Super I) filtriert. Das Lösungsmittel wurde abrotiert und man erhält 2,54 g (8,8 mmol) eines nahezu farblosen, öligen Produktes. Es handelt sich um ein Mischprodukt aus cis- und trans-Isomeren des 5-Allyloxy-2,2-dimethyl-1-(phenyl)methylenindan im Verhältnis 10:1. Die Auftrennung der Isomere erfolgte mittels Säulenchromatographie (Aluminiumoxid Aktivitätsstufe Super I/Petrolether). Der R_{f} des cis Isomers betrug 0,21.

### Beispiel 2

### Copolymer D4H + ABdhchol, ABB und 5-Allyloxy-2,2-dimethyl-1-(phenyl)methylenindan

0,41 g (1,70 mmol) Tetramethylcyclotetrasiloxan (D4H), 1,02 g (3.09 mmol) 4-(Propen-2-oxy) benzoesäure-4'-phenylphenylester (ABB), 1,70 g (3,10 mmol) 4-(Propen-2-oxy) benzoesäuredihydrocholesterylester (ABdhchol) und 0,20 g (0,69mmol) 5-Allyloxy-2,2-dimethyl-1-(phenyl)methylenindan wurden in 10 ml trockenem Toluol gelöst und nach Zugabe von 0,03 ml einer Lösung von Dicyclopentadienplatindichlorid (1 Gew.-% in Methylenchlorid) 1 h auf 100°C erwärmt.

Nach beendeter Reaktion wurde der Katalysator über eine kurze, mit Kieselgel gefüllte Säule (1 = 3 cm, Durchmesser = 3 cm) abgetrennt und das Produkt in Ethanol ausgefällt, bis der Restmonomergehalt unter 1 % betrug.

Das Endprodukt wurde über einen 0,2 µm-Filter filtriert und im Vakuum bei 90°C getrocknet. Man erhielt 2,8 g (84%) einer Substanz mit einer Reflexionswellenlänge bei 1130 nm. Die Substanz besitzt eine cholesterische Phase zwischen dem Glaspunkt bei 50°C und dem Klärpunkt bei 181°C.

### Beispiel 3

### Bestrahlung von 5-Allyloxy-2,2-dimethyl-1-(phenyl)methylenindan

0,352 mg cis 5-Allyloxy-2,2-dimethyl-1-(phenyl)methylenindan wurden in Methanol gelöst und in einem Meßkolben auf 10 ml verdünnt. Diese Lösung wurde in einer Quarzküvette mit dem Licht eines XeCl-Lasers (λ=308 nm) bestrahlt. Nach 10000 Pulsen (6mJ/cm²) wurde von dieser Lösung ein NMR-Spektrum gemessen. Aus dem Spektrum errechnet sich ein Anteil des trans-Isomeren von 13%. Signale für Cyclisierungs- oder Dimerisierungsprodukte wurden nicht beobachtet.

### Beispiel 4

### Herstellung eines strukturierbaren optischen Elements

Die Substanz aus Beispiel 2 wurde bei 120°C auf eine Glasplatte aufgetragen und mit einer zweiten Glasplatte abgedeckt; durch Scheren der Glasplatten erzeugt man einen dünnen Film dieser Substanz zwischen den Glasplatten. Die Probe wurde auf Raumtemperatur abgekühlt.

## Patentansprüche

1. Materialien der Formel I: 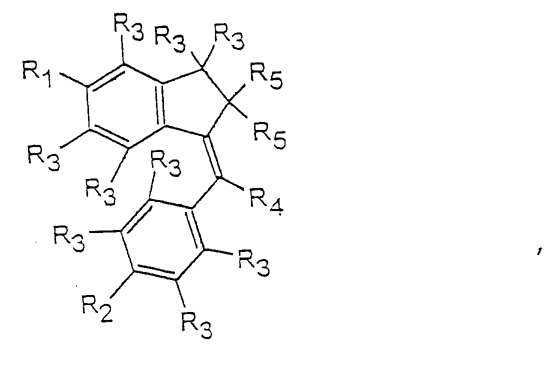 wobei
R₁, R₂, und R₃ jeweils unabhängig voneinander gleich oder verschieden sein können und Wasserstoffatom, Halogenatom, Hydroxygruppe, Nitrogruppe, Cyanogruppe oder einwertiger, gegebenenfalls substituierter organischer Rest bedeuten und
R₄ Wasserstoffatom, Halogenatom, Nitrogruppe, Cyanogruppe oder einwertiger, gegebenenfalls substituierter organischer Rest bedeuten und
R₅ gleich oder verschieden sein kann und Halogenatom, Hydroxygruppe, Nitrogruppe, Cyanogruppe, oder einwertiger, gegebenenfalls substituierter organischer Rest bedeutet.

2. Materialien gemäß Anspruch 1, dadurch gekennzeichnet, daß R₁, R₂, R₃ jeweils unabhängig voneinander Wasserstoffatom, Alkyl-, Alkenyl-, Alkoxy- oder Alkenoxyrest,
R₄ Wasserstoffatom oder Alkylrest,
R₅ Alkylrest bedeutet.

3. Materialien gemäß Anspruch 1 dadurch gekennzeichnet, daß
R₁ Alkenoxyrest
R₂ Wasserstoffatom, Alkyl- oder Alkoxyrest
R₃ Wasserstoffatom
R₄ Wasserstoffatom
R₅ Methylrest bedeutet.

4. Verfahren zur Herstellung von Materialien gemäß Anspruch 1, dadurch gekennzeichnet, daß ggf. substituierte 1-Indanone mit Alkylhalogeniden alkyliert werden, danach in einer Grignardreaktion mit ggf. substituierten Benzylmagnesiumhalogeniden umgesetzt werden und anschliessend sauer aufgearbeitet werden.

5. Polymere, welche mindestens einen Stilbenrest der Formel I aufweisen, bei dem einer der einwertigen Reste R₁ bis R₅ gegen einen zweiwertigen Rest ausgetauscht ist.

6. Polymere gemäß Anspruch 5, dadurch gekennzeichnet, daß es sich um ein Seitenkettenpolymer mit flüssigkristallinen Eigenschaften handelt.

7. Polymere gemäß Anspruch 5 oder 6, dadurch gekennzeichnet, daß das Polymerrückgrat aus cyclischen und/oder linearen Polysiloxanen aufgebaut ist.

8. Polymere gemäß Anspruch 5, dadurch gekennzeichnet, daß das Polymerrückgrat aus einem oder mehreren der Monomere, ausgewählt aus der Gruppe Acrylate, Methacrylate, Chloracrylate, Cyanacrylate, aufgebaut ist.

9. Polymere gemäß Anspruch 5, dadurch gekennzeichnet, daß das Polymer aus einem oder mehreren der Monomere, ausgewählt aus der Gruppe Vinylether, Vinylester, Styrol, α-Methylstyrol aufgebaut ist.

10. Optische Elemente, enthaltend Materialien gemäß einem oder mehreren der Ansprüche 1 bis 3 oder mindestens ein Polymer gemäß einem oder mehreren der Ansprüche 5 bis 9.
